# EUROPEAN PATENT APPLICATION

(11) **EP 4 439 581 A1**
(43) Date of publication of application: **02.10.2024**
(21) Application number: 23164669.6
(22) Date of filing: 28.03.2023
(51) Int. Cl.: G16H 40/20, G16H 10/60, G16H 50/30

(54) **ESTIMATION OF THE LENGTH OF STAY OF A PATIENT**

(71) Applicant: Ascom Ums Srl Universonale, 50018 Scandicci (FI) (IT)
(72) Inventor: CORDOS, Ariana-Anamaria, 400338 Cluj-Napoca - Jud. Cluj (RO); BURCHIETTI, Paolo, 50142 Firenze (IT)
(74) Representative: Bardehle Pagenberg Partnerschaft mbB Patentanwälte Rechtsanwälte

(57) **Abstract**

The invention relates to a computer-implemented method for estimating the length of stay, LOS, in a medical treatment facility of a patient receiving medical treatment and to a system, an apparatus and a computer program, configured to the perform the computer-implemented method. The computer-implemented method comprising: obtaining medical information associated with the patient, including information on one or more alarms triggered by a medical device; estimating, based on information on the one or more alarms triggered by the medical device, the length of stay of the patient.

## Description

### Field of the invention

The present disclosure relates to a computer-implemented method, an apparatus, and a computer program for prediction of the length of stay of a patient in a medical facility.

### Background

During the COVID 19 pandemic, it has shown that distributing patients effectively is crucial for providing the best possible health care for individual patients. The most critical patients of the hospital are located in the Intensive Care Unit (ICU). A commonly used metric for estimating the quality-of-care is the length of stay (LOS) in the ICU. Typically, the ICU LOS is used retrospectively to assess ICU efficiency or to understand the reasons for a patients long LOS and the corresponding elevated costs of care. However, there is a desire to be able predict the LOS of a patient beforehand to distribute patients in the hospital more effectively which allows to balance the occupancy of the ICU or other areas of the hospital. Predicting the LOS early also allows to estimate the potential costs associated with individual patients more precisely. Being able to estimate the occupancy and the cost of treatment beforehand is of increasing interest for multiple stakeholders, such as patients, families, financial backers of the hospital and ICU personnel.

In recent approaches, the LOS has been estimated extensively using models that consider a patient's weight, height, age, gender, and comorbidities. In "J. A. Gonzalez-Nóvoa, L. Busto, et. Al., *"Using Explainable Machine Learning to Improve Intensive Care Unit Alarm Systems".* Usig this information, the LOS of patients were estimated by analyzing vital signs throughout the hospitalization period. According to "H. Meissen, M. Gong, et. Al., *"The Future of Critical Care: Optimizing Technologies and a Learning Healthcare System to Potentiate a More Humanistic Approach to Critical Care"* each ICU patient generates over 1,000 data points per day, in addition to conventional vital signs, which are fed into clinical information technology solutions used to predict the LOS.

However, considering such large amounts of data points is complex and requires expensive infrastructure for time-consuming computations. Since hospitals are often provided with only a limited budged for high-end IT infrastructure, an effective and cheap method for estimating the LOS is desired.

Against this background, an object of the present invention is to provide an effective, comparatively cheap method for estimating the LOS of patients in a medical facility treating the patients, to ensure that occupancy of medical facilities and devices is optimized, and treatment of patients can be improved.

### Summary of the invention

The above-mentioned problem is at least partly solved by a computer-implemented method according to claim 1, by an apparatus according to claim 13, by a computer program according to claim 14, and by a vehicle according to claim 15.

A first aspect of the invention is directed to a computer-implemented method for estimating the length of stay, LOS, in a medical treatment facility of a patient receiving medical treatment, the method comprising obtaining medical information associated with the patient, including information on one or more alarms triggered by a medical device; estimating (predicting), based on information on the one or more alarms triggered by the medical device, the length of stay of the patient.

Considering alarms triggered by medical devices allows to estimate the severity of a patient's health state, as a more severe health state typically trigger more alarms, and a less severe typically trigger less alarms. Furthermore, by considering the information on the one or more alarms for estimating the LOS, a large number of data points can be disregarded which makes estimation of the LOS more efficient. This allows to estimate the LOS without having to invest in large IT infrastructure.

In another aspect, the LOS is estimated based on the information on the one or more alarms triggered by the medical device during an estimation interval, preferably within a predefined interval, preferably within the first 72h, more preferably within the first 48h, more preferably within the first 24h, even more preferably within the first 12h, and most preferably within the first 6h of the patient's medical treatment.

In a further aspect, the LOS of the patient is estimated based on the number of alarms occurring during the estimation interval.

Considering alarms triggered in the beginning of the treatment provides for the most accurate predictions, as this is a good indicator for treatments required to stability and care for the patient.

In yet another aspect, the one or more alarms associated with the patient are triggered based on one or more of one or more vital parameters of the patient, a predetermined timer running out, a predetermined setting of the medical device, and/or technical parameters of the medical device.

With the above settings and/or events, triggering an alarm can be adjusted according to the requirements set forth by the caregiver. For example, if it is determined that certain alarms cause imprecise predictions or certain other alarms provide for more precise predictions, triggering of these alarms can be adjusted.

In another aspect, the method further comprising updating the estimated LOS after receiving additional medical information.

Monitoring the further medical process of the patient and adjusting the estimated LOS accordingly allows to further improves the medical service for the patient. For example, if unexpected complications arise, it is desired to adjust the estimated LOS accordingly to be able to provide the best medical care for the patient.

In a further aspect, the medical information is obtained from a hospital support system, preferably one or more of a Clinical Decision Support System, CDSS, a billing data transferring format, ADT, system, a Laboratory system, a BGA system, an EHR/EMR system, and/or a PDMS system.

In another aspect, the method further comprising forwarding the medical information associated with the patient, and/or the estimated LOS to one or more devices and/or to a hospital information/support system.

Such medical information systems are commonly used and well established in a hospital environment. Since these systems already obtain large amounts of patient's data, it is convenient to obtain the medical information from these established systems to reduce the overall cost of implementing the above-described method. Similarly, the medical information obtained from the medical devices may also be forwarded to the respective hospital information/support systems.

In another aspect, the method further comprising collecting a plurality of LOS estimates associated with a plurality of patients receiving medical treatment; predicting a future occupancy of the medical facility treating the plurality of patients based on the estimated LOS information associated with the plurality of patients.

With the above, it is possible to obtain a precise estimate of a future occupancy of the medical facility treating the patients. This makes it possible to optimize admission of patients into the medical facility. For example, if it is estimated that the occupancy of the medical facility reaches a certain threshold, approaching ambulances may be redirected to another medical facility.

In another aspect, the method further comprising forwarding information related to the estimated LOS to a hospital information/support system.

In a further aspect, the method further comprising triggering and/or displaying a notification on an electronic device including information related to the estimated LOS, preferably the electronic device being associated with a hospital information/support system.

In yet another aspect, the notification further includes information related to the estimated future occupancy.

In a further aspect, the method further comprising generating a report based on previous, current and/or future estimated LOS and/or occupancy of the medical facility.

Providing a report including the information of estimated LOS allows to identify discrepancies between the actual LOS of a patient and the estimated LOS of the corresponding patient. Moreover, the report may be used to equip and organize the hospital more optimally in a long-term manner.

In a further aspect, the medical device is a patient monitor, a mechanical ventilator, patient respiratory ventilator, an infusion pump, a dialysis machine or other medical device used for medical treatment and/or monitoring of a patient, preferably connected to a hospital information system.

Another aspect of the invention is directed to an apparatus, preferably a mobile apparatus, comprising means configured to perform the above-described method.

By performing the above-described method using a mobile apparatus, the hospital staff may obtain the estimated LOS at any time irrespective of their current location. This allows individual hospital staff to plan their individual tasks more efficient.

A further aspect of the invention is directed to a system comprising one or more medical devices configured to generate an alarm based on an event associated with a patient; and the apparatus configured to perform the above-described method.

Yet another aspect of the invention relates to a computer program comprising instructions, which when executed by a computing system, causing the computing system to perform the above-described method.

### Brief description of the drawings

Various aspects of the present invention are described in more detail in the following by reference to the accompanying figures without the present invention being limited to the embodiments of these figures.
- Fig. 1: illustrates an exemplary method for estimating the LOS of a patient;
- Fig. 2: illustrates an exemplary system used for estimating the LOS of the patient;
- Fig. 3: illustrates the training of a machine learning model for estimating the LOS of the patient;
- Fig. 4: illustrates inference of the trained machine learning model for estimating the LOS of the patient;
- Fig. 5: illustrates updating the estimated LOS after receiving additional medical information, using the trained machine learning model;
- Fig. 6: shows LOS estimation results associated with patients using the trained machine learning model;
- Fig. 7: shows a medical system for monitoring a medical facility.

### Detailed description

In the following, aspects of the present invention are described in more detail with reference to the figures.

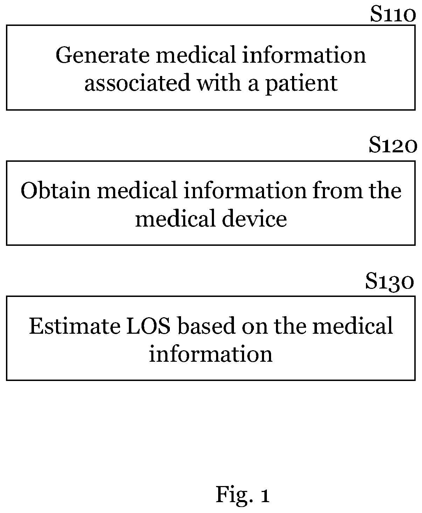

Figure 1 shows the prediction of the LOS of a patient according to an embodiment. In step S110, one or more medical devices 101 generate medical information associated with a patient. The patient being a person receiving a medical service in a medical facility. A computing device 110 obtains (S120) the medical information from the medical device and estimates (S130) a length of stay (LOS) associated with the patient based on the medical information. The estimated LOS may be updated (S140) (not shown) after receiving additional medical information. A plurality of LOS estimates associated with a plurality of patients receiving medical treatment may be collected and a future occupancy of the medical facility treating the plurality of patients may be determined based on the estimated LOS information associated with the plurality of patients (S150) (not shown). Alternatively or additionally, a notification may be triggered and/or displayed (S160) (not shown) on an electronic device, such as a mobile device 160, including information related to the estimated LOS.

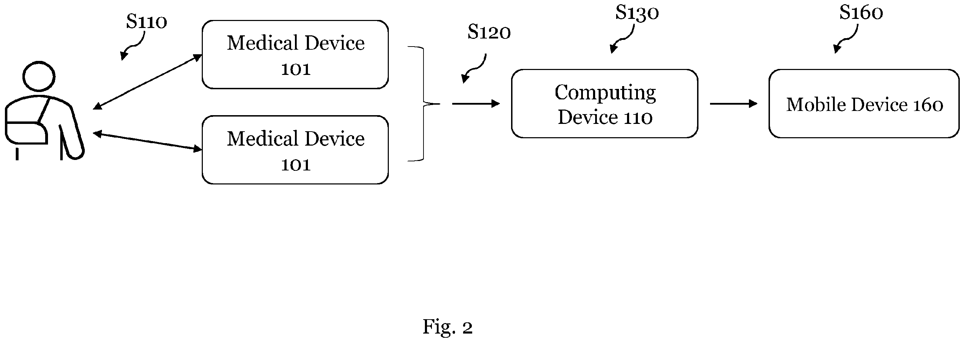

Fig. 2 shows the above prediction of the LOS associated with a patient in more detail.

One or more medical devices 101 are associated with the patient. Each of the one or more medical devices 101 associated with the patient generate the medical information associated with the patient (S110). The one or more medical devices 101 may be any medical device that is used for therapeutic and/or diagnostic measures in a medical facility. For example, the one or more medical devices 101 may be a patient monitor, a mechanical ventilator, a patient respiratory ventilator, an infusion pump, a dialysis machine or other medical device used for medical treatment and/or monitoring of a patient. Preferably the one or more medical devices 101 are connected to a hospital information system (HIS), such as a Clinical Decision Support System (CDSS). Moreover, the medical information associated with the patient may be provided by one or more another medical devices 102 that do not generate the medical information associated with the patient. For example, the one or more another medical devices 102 may be configured to distribute and/or store medical information associated with the patient, preferably obtained by the one or more medical devices 101. Moreover, the one or more medical devices 101 may be configured to distribute/store medical information associated with the patient, the medical information being generated at the respective one more medical devices, or at another one of the one or more medical devices 101. In this case, the one or more medical devices 101 may be configured to function as a repeater and/or router and/or server and/or storage for the another one of the one or more medical devices 101.

The medical information associated with the patient may comprise any medical information associated with the patient that is generated and/or handled in the medical facility. For example, the medical information may comprise diagnostic information, such as monitoring information and/or laboratory results obtained from one or more monitoring/diagnostic medical devices. Additionally or alternatively, the medical information may comprise therapeutic information, such as settings of the one or more medical devices 101 configured to support therapeutic measures associated with the patient and/or corresponding measurement results obtained from the one or more medical devices associated with therapeutic measures associated with the patient.

The one or more medical devices 101 may be configured to generate an alarm associated with the patient based on one or more predefined events. For example, the one or more medical devices 101 may be configured to generate an alarm based on one or more of a characteristic of the medical information, such as a vital parameter of the patient, a predetermined timer running out, a predefined event, a predetermined setting of the medical device, and/or technical parameters of the medical device. The characteristic of the medical information may indicate that a value of the medical information has reached a certain threshold value, that a value of the medical information indicates a predefined type of value and/or any other characteristic aspect associated with medical information. For example, if a patient monitor indicates that the heartrate of the patient is above/below a predefined threshold, an alarm may be triggered. Similar predefined thresholds may be configured for any other of the above mentioned exemplary one or more medical devices 101 used in the medical facility, such as the mechanical ventilator, the patient respiratory ventilator, the infusion pump, the dialysis machine or the other medical device used for medical treatment and/or monitoring of a patient. In another example, an alarm may be generated based on an aspect of the medical information representing a certain type of information. For example, if the blood type of the patient was tested in a laboratory, and the test results indicate that the patient's blood has a certain type, an alarm may be triggered based on the test results. Additionally or alternatively, the one or more medical devices 101 may be part of a communication network and configured to generate an alarm based on the medical information being transmitted through this network. In this example, the one or more medical devices 101 may be configured to function as a network repeater and/or as a network monitor.

To facilitate handling of the respective information, the information on the one or more alarms triggered by the one or more medical devices 101 is part of the medical information associated with the patient.

The information associated with the one or more alarms may be transmitted from and/or stored on the one or more medical devices 101. For example, the one or more medical devices 101 may count the number of alarms generated in a certain time interval and store the information associated with the number of alarms generated in the certain time interval in a storage. Preferably, the information on the one or more alarms triggered by the one or more medical devices 101 is stored during an estimation interval, preferably within a predefined interval, preferably within the first 72h, more preferably within the first 48h, more preferably within the first 24h, even more preferably within the first 12h, and most preferably within the first 6h of the patient's medical treatment. The storage may be implemented in the one or more medical devices 101 and/or in an additional storage facility, such as a local storage facility in the medical facility and/or in a cloud storage/network storage. In another example, the one or more medical devices 101 may transmit the information associated with the alarm, preferably with the number of alarms, to another of the one or more medical devices 101, or to a computing device 110 configured to estimate the LOS of the patient based on the medical information. In addition or alternatively, the information may also be transmitted to a hospital information/support system (HIS). Exemplary hospital support systems are one or more of a Clinical Decision Support System, CDSS, a billing data transferring format, ADT, system, a Laboratory system, a Blood Gas Analysis, BGA, system, an electric health record, EHR, system, an emergency medical services, EMS, system, and/or a patient data management, PDMS, system. Such systems may also serve as a central storage and/or distribution facility for the medical information and the medical information including the information associated with the one or more alarms. For example, the computing device 110 configured to estimate the LOS of the patient based on the medical information may obtain the medical information from one such a hospital information/support system.

Any information transmitted in any of the examples may be transmitted in real-time or in predefined intervals. While transmitting data in real-time allows estimating and/or monitoring the LOS of individual patients in real-time, transmitting the data in predefined intervals further reduces the network/computing load.

In the embodiment shown in Fig. 2, the computing device 110 obtains the medical information including information on one or more alarms triggered directly from the one or more medical devices 101 (S120).

Preferably, the information on the one or more alarms triggered by the one or more medical devices 101 is obtained during the estimation interval, preferably within the predefined interval, preferably within the first 72h, more preferably within the first 48h, more preferably within the first 24h, even more preferably within the first 12h, and most preferably within the first 6h of the patient's medical treatment. However, the information on the one or more alarms triggered by the one or more medical devices 101 may also be obtained at any later point in time. For example, in some embodiments, the one or more medical devices 101 store the information on the one or more alarms triggered by the one or more medical devices 101 for at least the estimation interval and transmits the information after the information has been stored/collected for the estimation interval. As outlined above, the computing device 110 may obtain the medical information including information on the one or more alarms triggered by the one or more medical devices 101 in real-time or in certain predefined time intervals. Whether the information ins transmitted in real-time or in predefined intervals may be defined in a transmission rule. The transmission rule may be predefined or changed dynamically based on the current network load and/or computing load of the computing device 110 and/or the communication network associated with the computing device 110. In one example, the computing device 110 may monitor the current network load/computing load and determine an optimal current transmission rule based on this information and change the current transmission rule based on the determined transmission rule.

Based on the medical information including information on the one or more alarms triggered by the one or more medical devices 101, the computing device 110 estimates the LOS associated with the patient (S130).

For example, a certain number of alarms triggered in the estimation interval may be indicative of a certain LOS associated with the patient. That is, a relatively high number of alarms triggered in the estimation interval may be indicative of a relatively high LOS associated with the patient, wherein a relatively low number of alarms triggered in the estimation interval may be indicative of a relatively low LOS associated with the patient. In addition, certain weight factors may be associated with some of the one or more alarms. For example, alarms associated with respiratory ventilators may receive a larger weight factor than alarms associated with the passing of amount of time. Accordingly, typically, the LOS associated with the patient is high if the number of alarms having a large weight factor is high, and the LOS associated with the patient may be low even if the total number of alarms is relatively high, if the number of alarms associated with a large weight factor is low.

In some embodiments, the LOS may be determined such that alarms occurring and/or being obtained in certain intervals of the estimation interval are weighted in a manner such that alarms occurring in the certain intervals are weighted stronger (have more impact on the estimated LOS associated with the patient) then alarms occurring/being obtained in another certain interval. For example, alarms occurring in the first 12 hours of the treatment of the patient may be weighted more such that these alarms have more impact on the LOS associated with the patient. This approach can be beneficial if information related to the initial treatment of the patient is of particular interest. Similarly, alarms occurring at a later stage of the patient's treatment, such as after 24h or 48h may be weighted more, such that alarms occurring at the later stage have more impact on the LOS associated with the patient. This can be beneficial if information related to the subsequent treatment (after the initial treatment of the patient) is of particular interest.

Furthermore, in some embodiments, the LOS associated with the patient is estimated by considering previous alarms generated/obtained in a previous estimation interval. For example, if it is determined that in a first estimation interval a certain number/type of alarms were triggered and in a second estimation interval (in a timely manner) after the first estimation interval a certain number/type of alarms are triggered, the LOS may be estimated by considering both numbers/types of alarms of both estimation intervals. For example, if a large number of alarms and/or a large number of alarms having a large weight factor occurring in the first estimation interval are followed by a small number of alarms/alarms having small weight factors in the second estimation interval, the LOS associated with the patient may be estimated as being relatively low. That is, a high number of alarms/alarms having large weight factors in the first estimation interval followed by a small number of alarms/alarms having a small weight factor in the second estimation interval may be indicative of a significant/rapid improvement of the health status of the patient. However, if a certain number of alarms/alarms having a certain weight factor in the first estimation interval are followed by a similar certain other number of alarms/alarms having a similar certain weight factor in the second estimation interval, it may be determined that the health status of the patient does not improve significantly/rapidly and the LOS may be associated as relatively high. Naturally, if the number of alarms/alarms having large weight factors increase, the LOS may be estimated as being relatively high, as the health status of the patient appears to deteriorate.

In the above examples, the LOS is estimated based on the information on the one or more alarms triggered by the one or more medical devices 101. However, to improve estimation of the LOS associated with the patient, certain additional information associated with the patient may also be considered to improve the estimation of the LOS associated with the patient. The certain additional information associated with the patient may include one or more of the age of the patient, the gender of the patient, the marital status of the patient, previous medical conditions of the patient, the weight of the patient, the size of the patient and any other information that could have an impact on the LOS associated with the patient.

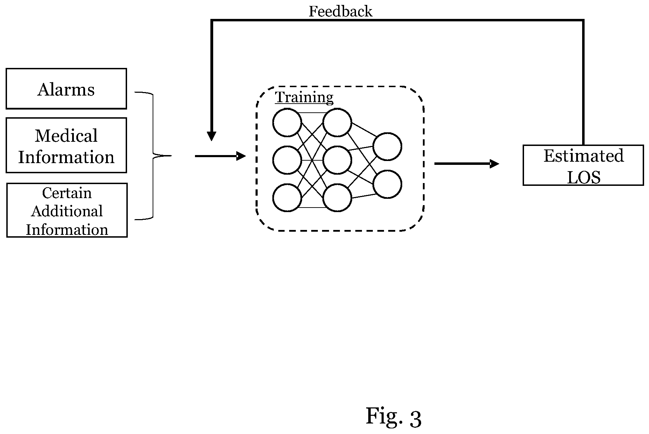

In Figs. 3 to 5, a machine learning model-based approach for estimating the LOS is illustrated that considers the above as parameters for the machine learning model.

For estimating the LOS, regression or classification models as particularly well suited. Typically, the former will provide a numerical value indicating the number of days a particular patient will spend in the medical facility, wherein the latter will provide an interval of days the patient is expected to stay in the medical facility.

In Fig. 3, the training of the machine learning model is illustrated. As outlined above, certain time intervals and types of alarms may be associated with a certain weight factor. These weight factors may be implemented in the training stage of the machine learning model and adjusted during the training to obtain the best results. Similarly, while estimating the LOS based on the number of alarms obtained in the estimation interval has shown good results, the certain additional information associated with the patient may also be included in the training stage with corresponding weigh factors. Moreover, aspects such as the transmission rule associated with the computing device 110 may also be considered by the model. The estimated LOS may then be compared to actual LOS corresponding to the input parameters and the results may then be fed back into the model for training.

The machine learning model may be trained by the computing device 110 or by an external system that provides the trained machine learning model to the computing device 110.

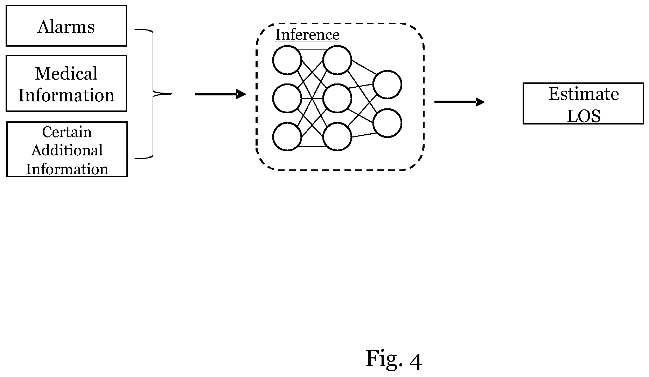

During inference, as shown in Fig. 4, the computing device 110 may then estimate the LOS associated with the patient based on the above-described input parameters including the information on the one or more alarms triggered by the one or more medical devices 101. Preferably, the computing device 110 using the trained machine model estimates the LOS using the machine learning model during the estimation interval, preferably within a predefined interval, preferably within the first 72h, more preferably within the first 48h, preferably within the first 24h, even more preferably within the first 12h and most preferably within the first 6h of the patient's medical treatment. During inference, the LOS may be estimated using the information related to the one or more alarms and one or more of the medical information associated with the patient (not including the information on the one or more alarms) and/or the certain additional information.

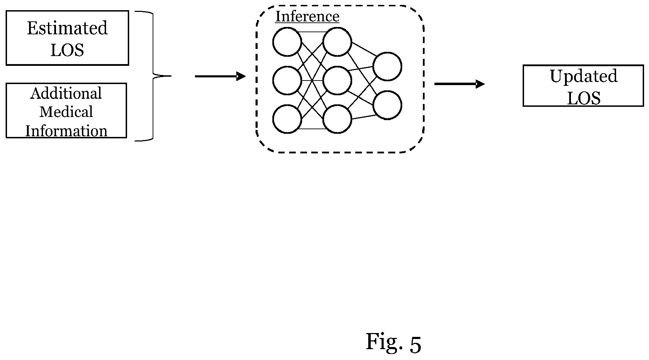

During inference, after the estimation of the LOS of the patient, the estimation of the LOS may be updated/adjusted if additional medical information is obtained after the training of the machine learning model, as shown in an example in Fig. 5. For example, if, after the estimation interval, additional medical information associated with the patient indicates improvement/worsening of the health status of the patient, the LOS may be estimated considering this additional information. For example, if a large number of alarms are triggered after the estimation interval, the estimated LOS may increase based on the large number of alarms indicating a worsening of the patient's health status. Similarly, if additional medical information associated with the patient indicates improvement of the health status of the patient, for example if inflammatory parameters indicate that an inflammation has ended, the estimated LOS may be reduced. Accordingly, updating/adjusting the estimated LOS based on additional medical information allows to improve estimation of the LOS associated with the patient even if the health status changes significantly at a later point in time.

Preferably, the LOS is estimated for each of a plurality of patients receiving medical treatment at the medical facility. The plurality of estimated LOS associated with the plurality of patients may be collected and used to predict a future occupancy of the medical facility treating the plurality of patients. Moreover, based on this information, a report may be generated including previous, current and/or future estimated LOS and/or estimated occupancies.

The estimated LOS and/or estimated future occupancy and/or reports associated with the LOS and/or the occupancy may be transmitted from the computing device to an external device, such as a mobile device 160 (S160).

For example, past occupancies and the respective estimated LOS of the corresponding patients may be used to train a further machine learning model that provides estimates of the future occupancy of the medical facility in an improved, precise manner. The further machine learning model may be trained using information related to the past occupancy of certain areas of the medical facility as well as information related to the estimated LOS of the corresponding patients occupying the areas of the medical facilities.

A machine learning model trained in this manner can be used to estimate the future occupancy of certain areas of the medical facility by patients can be determined. For example, if the current occupancy is high and the estimated LOS is large for a large number of patients currently occupying the respective medical facilities, it may be predicted that the occupancy of the respective medical facilities will also be high in a near future.

This information may be used to redirect new arriving patients to certain other areas of the medical facility or to other medical facilities, such that extensive overload of the certain areas is avoided. Moreover, if it is determined that patients will occupy areas of the medical facility for a relatively long period, this information can be used to adjust planning of the areas accordingly. For example, certain medical devices or facilities may be added to the respective medical facilities to avoid overload of these areas of the medical facility.

The report may be used to improve planning of the medical facility in the future. Preferably, reports of a plurality of medical facilities, such as individual hospitals, are compared to indicate whether some areas of the respective medical facilities need improvement, and to detect local anomalies. For example, the report may indicate that the LOS of patients in the ICU of a first hospital are significantly higher than the LOS of patients in the ICU of other hospitals in a similar geographic area. This could indicate that there is an issue in the ICU of the first hospital that needs adjustment. For example, the difference in the estimated LOS associated with the first hospital may indicate that medical treatment in the ICU of the first hospital is worse than in the others and that, for example, more staff, training and or medical devices are required.

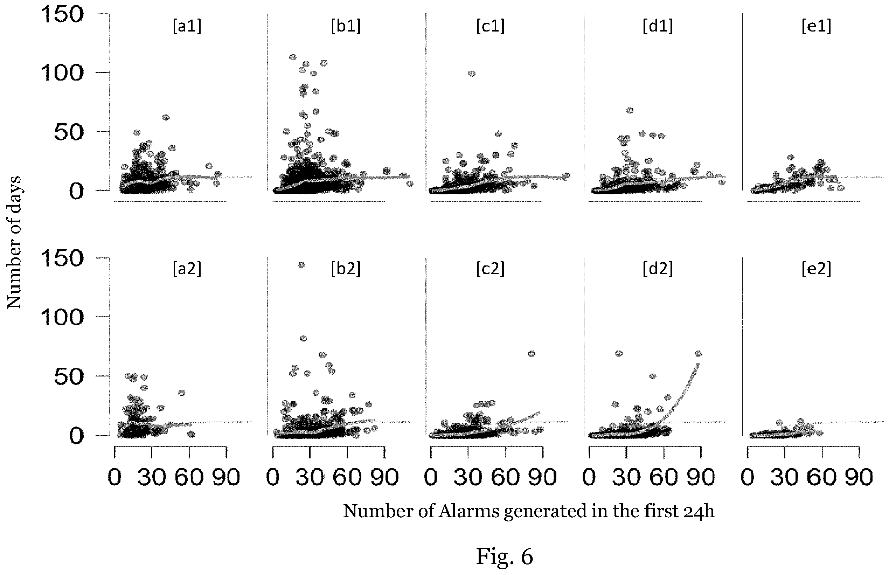

Fig. 6 shows experimental LOS estimations results using a machine learning model for estimating the LOS. In this example a regression model was trained using data of patients in an ICU for estimating the corresponding LOSs. In the graphs shown in Fig. 6, the number of alarms in the first 24h (x-axis) are plotted against the number of days (y-axis), wherein the grey dots represent the estimated LOS of individual patients. In each of the graphs [a1] to [e2], the thick grey line represents the predicted LOS of the patients, and the thin grey line represents the actual LOS of the corresponding patients, wherein the entire data set was used to generate the thin grey line. The upper row of graphs ([a1] - [e1]) corresponds to patients that have received a surgical intervention, and the bottom row of graphs ([a2] - [e2]) correspond to patients that visit the medical facility without surgical intervention.

In each of the graphs, certain additional information associated with the patient has been considered in addition to the information on the one or more alarms triggered by the medical device(s). That is, graphs [a1] and [a2], the LOS of newborn patients have been estimated. In graphs [b1] and [b2], the LOS of lactating mothers have been estimated. In graphs [c1] and [c2] the LOS of paediatric patients have been estimated. In graphs [d1] and [d2] the LOS of teenagers have been estimated, and in graphs [e1] and [e2], the LOS of adult patients have been estimated.

As can be seen in the graphs of Fig. 6, estimating the LOS using machine learning has shown good results. In particular with regards to the patients having received surgical intervention (the upper row of graphs). While there are graphs that show some anomalies, in most cases the estimated LOS was relatively close to the LOS. Additionally, the above shows that for patients not having received surgical intervention, it could be beneficial to train the model differently and use a first machine learning model for estimating the LOS of patients having received surgical intervention and train a second machine learning model for estimating the LOS of patients not having received surgical intervention. Nevertheless, applying the machine learning model has confirmed a degree of correlation between the number of alarms within the first hours of the patient in the medical facility and the total number of hours spent in the facility (the LOS).

Most significantly, further experimental results have shown that considering the number of alarms within the first 6 hours of treatment of the patient can already provide reasonable LOS predictions in terms of sensitivity and specificity. Nevertheless, as outlined above, considering a longer interval and more types of data allow to increase the sensitivity and specificity of the estimated LOS.

Furthermore, as shown above, in some embodiments it may be preferred to use specifically trained machine learning model for estimating the LOS associated with patients having specific emergencies. That is, it may be preferred to train a plurality of individual machine learning models for a plurality of patients having emergencies in different medical fields. Such as first machine learning model for a first group of patients having medical emergencies in a first medical field, and a second machine learning model for a second group of patients having medical emergencies in a first medical field. For example, it may be preferred to train and apply a first machine learning model for estimating the LOS of patients having received surgical intervention and to train and apply a second machine learning model for estimating the LOS of patients not having received surgical intervention. Similarly, it may be preferred to train a first machine learning model for patients receiving treatment in a first medical field (such as a dental emergency) and a second machine learning model for patients treated in a second medical field (such as a pulmonary emergency).

Similarly, considering the above example with the surgical intervention, additionally or alternatively, individual machine learning models may be trained/applied for estimating the LOS of corresponding different kinds of medical treatments. That is, a first model May be trained for a relatively invasive surgery (such as a hearth transplant), and a second model may be trained for a relatively non-invasive surgery (such as an appendectomy). In as similar manner, the models may also be trained and applied with respect to emergencies associated with certain body parts/organs.

### Exemplary implementation of the above-described estimation of the LOS

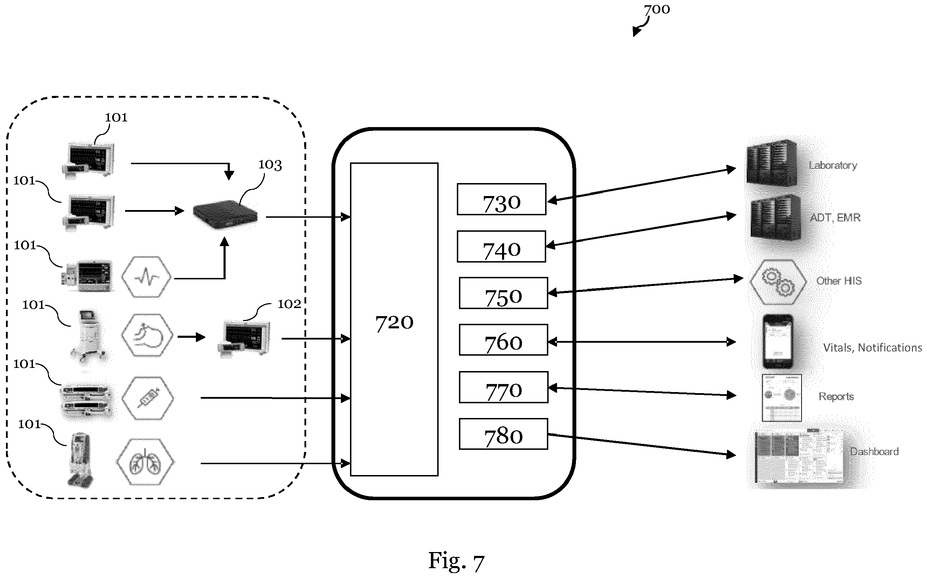

In Fig. 7, a medical system 700 for monitoring the medical facility is shown, according to an embodiment. Th The medical system 700 is configured to estimate the LOS associated with a patient, similar to any of the above-described methods. In this embodiment, the medical system 700 comprises one or more medical devices 101, a computing device 710 and one or more external devices 790, receiving information from the computing device 710. The computing device 710 may be similar to the computing device 110, described in the previous embodiments. Preferably, the computing device 710 may also receive medical information associated with the patient from the one or more external devices 790. The one or more medical devices 101 are configured to generate and/or provide medical information associated with a patient. In this embodiment, the computing device 710 is configured to obtain the medical information generated by the one or more medical devices 101 through a communication means 720. The communication means includes means for receiving the medical data associated with the patient directly from the one or more medical devices 101, or indirectly via a server infrastructure, a communication hub, such as a router 103, or another medical device 102 functioning as a gateway/repeater. The communication means 720 is configured to receive the medical information via wired communication connection or a wireless communication connection.

Based on the received medical information associated with the patient, and any other type of information described above, the computing device 710 estimates the LOS associated with the patient. In the example shown in Fig. 7, one computing device 710 is associated with the medical facility. In this case, the computing device 710 estimates the LOS for each patient in the medical facility. In other examples, a plurality of computing device 710 are associated with medical facility. In such examples, the one or more computing devices 710 may be associated with a certain number of patients. For example, one or more computing devices 710 may be associated with only one area of the medical facility. In some examples, one or more computing devices 710 may be used only for training the machine learning model, wherein one or more other computing devices 710 may be used only for applying (inference) the trained machine learning model. Furthermore, in further examples, one computing devices 710 may be associated with a plurality of medical facilities.

After the computing device 710 has estimated the LOS associated with the patient, the computing device 710 may transmit the information related to the estimated LOS, and preferably also further information associated with the corresponding patient, to an external device 790. The external device 790 may be one or more of a server, a mobile device 160, a display device, a laboratory, another computing device 710 associated with another medical facility and other potential external stakeholders having interest in such information.

For the data exchange to the external device 790, the medical device 710 may further comprise one or more of a lab gateway 730, an EMR gateway 740, an other-HIS gateway 750, a mobile server 760, means for LOS and benchmarking reports 770, and a dashboard server 780. The lab gateway 730 is configured to transmit/receive medical information, such as laboratory results, associated with the patient to/from the laboratory. The EMR gateway 740 is configured to transmit/receive information related to an admission, discharge, and transfer (ADT) system and/or an electronic medical record system (EMR). The another-HIS gateway 750 is configured to exchange information with another hospital information system (HIS). The another HIS may be allocated in the same medical facility as the computing device 710, or in a remote medical facility. The mobile server is configured to transmit/receive information associated with the patient. For example, the mobile server may be configured to transmit and/or receive medical information associated with the patient to a mobile device. Alternatively or additionally, the mobile server may be configured to issue a notification associated with the patient and/or the occupancy of the medical facility at the mobile device. The means for LOS and benchmarking reports 770 is configured to transmit and/or receive reports associated with the LOS of the patient and/or with the occupancy of the medical facility to/from external entities. For example, the means for LOS and benchmarking reports 770 may be configured to transmit reports associated with a current occupancy and/or an estimated future occupancy and/or estimated LOS of one or more patients to external stakeholders, such as the board of the respective medical facility. This allows the external stakeholders to receive relevant information on the medical facility and consider options to improve the treatment of patients at the respective facilities, if the estimated LOS and/or occupancy indicates that there is a need for adjustment. The dashboard server 780 is configured to transmit information associated with the estimated LOS to an external dashboard configured to display the corresponding information.

The method according to the present invention may be implemented in terms of a computer program which may be executed on any suitable data processing device comprising means (e.g., a memory and one or more processors operatively coupled to the memory) being configured accordingly. The computer program may be stored as computer-executable instructions on a non-transitory computer-readable medium.

Embodiments of the present disclosure may be realized in any of various forms. For example, in some embodiments, the present invention may be realized as a computer-implemented method, a computer-readable memory medium, or a computer system.

In some embodiments, a non-transitory computer-readable memory medium may be configured so that it stores program instructions and/or data, where the program instructions, if executed by a computer system, cause the computer system to perform a method, e.g., any of the method embodiments described herein, or, any combination of the method embodiments described herein, or, any subset of any of the method embodiments described herein, or, any combination of such subsets.

In some embodiments, a computing device may be configured to include a processor (or a set of processors) and a memory medium, where the memory medium stores program instructions, where the processor is configured to read and execute the program instructions from the memory medium, where the program instructions are executable to implement any of the various method embodiments described herein (or, any combination of the method embodiments described herein, or, any subset of any of the method embodiments described herein, or, any combination of such subsets). The device may be realized in any of various forms.

Although specific embodiments have been described above, these embodiments are not intended to limit the scope of the present disclosure, even where only a single embodiment is described with respect to a particular feature. Examples of features provided in the disclosure are intended to be illustrative rather than restrictive unless stated otherwise. The above description is intended to cover such alternatives, modifications, and equivalents as would be apparent to a person skilled in the art having the benefit of this disclosure.

The scope of the present disclosure includes any feature or combination of features disclosed herein (either explicitly or implicitly), or any generalization thereof, whether or not it mitigates any or all of the problems addressed herein. In particular, with reference to the appended claims, features from dependent claims may be combined with those of the independent claims and features from respective independent claims may be combined in any appropriate manner and not merely in the specific combinations enumerated in the appended claims.

## Claims

1. A computer-implemented method for estimating the length of stay, LOS, in a medical treatment facility of a patient receiving medical treatment, the method comprising:
obtaining medical information associated with the patient, including information on one or more alarms triggered by a medical device;
estimating, based on information on the one or more alarms triggered by the medical device, the length of stay of the patient.

2. The method according to the preceding claim, wherein the LOS is estimated based on the information on the one or more alarms triggered by the medical device during an estimation interval, preferably within a predefined interval, preferably within the first 72h, more preferably within the first 48h, preferably within the first 24h, even more preferably within the first 12h and most preferably within the first 6h of the patient's medical treatment.

3. The method according to the preceding claim, wherein the LOS of the patient is estimated based on the number of alarms occurring during the estimation interval.

4. The method according to any one of the preceding claims, wherein the one or more alarms associated with the patient are triggered based on one or more of one or more vital parameters of the patient, a predetermined timer running out, a predetermined setting of the medical device, and/or technical parameters of the medical device.

5. The method according to any one of the preceding claims further comprising:
updating the estimated LOS after receiving additional medical information.

6. The method according to any one of the preceding claims, wherein
the medical information is obtained from a hospital support system, preferably one or more of a Clinical Decision Support System, CDSS, a billing data transferring format, ADT, system, a Laboratory system, a BGA system, an EHR/EMR system and/or a PDMS system.

7. The method according to any one of the preceding claims, further comprising:
collecting a plurality of LOS estimates associated with a plurality of patients receiving medical treatment;
predicting a future occupancy of the medical facility treating the plurality of patients based on the estimated LOS information associated with the plurality of patients.

8. The method according to any one of the preceding claims, further comprising:
triggering and/or displaying a notification on an electronic device including information related to the estimated LOS.

9. The method according to claims 7 and 8, wherein the notification further includes information related to the estimated future occupancy.

10. The method according to claim 7 further comprising:
generating a report based on previous, current and/or future estimated LOS and/or occupancy of the medical facility.

11. The method according to any one of the preceding claims, further comprising:
forwarding the medical information associated with the patient to one or more devices and/or to a hospital information system.

12. The method according to any one of the preceding claims, wherein the medical device is a patient monitor, a mechanical ventilator, patient respiratory ventilator, an infusion pump, a dialysis machine or other medical device used for medical treatment and/or monitoring of a patient, preferably connected to a hospital information system.

13. Apparatus, preferably a mobile apparatus, comprising means configured to perform the method of any one of claims 1 to 12.

14. A system comprising:
one or more medical devices configured to generate an alarm based on an event associated with a patient; and
the apparatus according to claim 13.

15. A computer program comprising instructions which when executed by a computing system, cause the computing system to perform the method of any of the claims 1 to 12.
